# EUROPÄISCHE PATENTSCHRIFT

(11) **EP 2 399 546 B1**
(45) Veröffentlichungstag und Bekanntmachung des Hinweises auf die Patenterteilung: **01.07.2015**
(21) Anmeldenummer: 10189952.4
(22) Anmeldetag: 04.11.2010
(51) Int. Cl.: A61C 19/00, A61B 19/00, A61L 2/18

(54) **Reinigungs- oder Pflegegerät für medizinische Instrumente**
Cleaning or maintenance device for medical instruments
Appareil de nettoyage ou d'entretien pour instruments médicaux

(30) Priorität: 28.06.2010 EP 10167445
(43) Veröffentlichungstag der Anmeldung: 28.12.2011
(73) Patentinhaber: W & H Dentalwerk Bürmoos GmbH, 5111 Bürmoos (AT)
(72) Erfinder: Pfaffinger, Nikolaus, 5120 St. Pantaleon (AT); Spieler, Christian, 5152 Michaelbeuern (AT)
(74) Vertreter: Benda, Ralf

(56) Entgegenhaltungen:
- EP-A1- 1 902 670
- WO-A1-94/15547
- US-A1- 2009 188 531

## Beschreibung

Die vorliegende Erfindung betrifft ein Reinigungs- oder Pflegegerät für medizinische Instrumente gemäß dem Oberbegriff des Anspruchs 1 und ein Verfahren zu dessen Herstellung.

Ein derartiges Reinigungs- oder Pflegegerät für medizinische Instrumente ist aus der Anmeldeschrift WO 94/15547 A1 bekannt worauf die zweiteilige Form des Anspruchs 1 basiert. Das Reinigungs- oder Pflegegerät umfasst einen Rahmen, der die zu reinigenden Instrumente umgibt und an dem mehrere Düsen vorgesehen sind, durch die ein Reinigungs- oder Pflegemedium auf die Außenseite der Instrumente gesprüht werden kann. Um die Instrumente entlang ihrer gesamten Länge mit dem Reinigungs- oder Pflegemedium beaufschlagen zu können, ist eine Gewindespindel vorgesehen, an welcher der Rahmen befestigt ist und die mittels eines Motors angetrieben wird, wodurch der Rahmen relativ zu den zu reinigenden Instrumenten bewegbar ist.

Der Nachteil dieses Reinigungs- oder Pflegegeräts liegt darin, dass die Gewindespindel in der Reinigungskammer angeordnet ist, wodurch Raum für die Aufnahme der zu reinigenden Instrumente verloren geht und / oder das Volumen der Reinigungskammer und damit des gesamten Reinigungs- oder Pflegegeräts vergrößert werden muss, um eine entsprechende Anzahl von zu reinigenden Instrumenten aufnehmen zu können. Des Weiteren behindert die Gewindespindel die Reinigung der Reinigungskammer.

Der vorliegenden Erfindung liegt daher die Aufgabe zugrunde ein Reinigungs- oder Pflegegerät für medizinische Instrumente zu schaffen, das einen Rahmen mit Düsen aufweist, der entlang einer Längsachse der zu reinigenden Instrumenten bewegbar ist, welches jedoch die genannten Nachteile nicht aufweist. Insbesondere soll das Reinigungs- oder Pflegegerät derart beschaffen sein, dass kein Raum für die Aufnahme der zu reinigenden Instrumente verloren geht oder die Reinigung des Reinigungs- oder Pflegeraums nicht behindert wird.

Zur Lösung dieser Aufgabe wird ein Reinigungs- oder Pflegegerät für medizinische Instrumente, insbesondere für dentale Instrumente, vorgeschlagen, das umfasst: Einen Reinigungs- oder Pflegeraum, zumindest eine im Reinigungs- oder Pflegeraum angeordneten Anschlussvorrichtung, an die ein zu reinigendes Instrument anschließbar ist, eine Fördervorrichtung zur Förderung zumindest eines Reinigungs- oder Pflegemediums, wobei die Fördervorrichtung einen Rahmen aufweist, an dem zumindest eine Düse zur Abgabe eines Reinigungs- oder Pflegemediums an die Außenseite des zu reinigenden Instruments vorgesehen ist, wobei der Rahmen relativ zur zumindest einen Anschlussvorrichtung bewegbar ist, um im Wesentlichen einer Längsachse eines an die zumindest eine Anschlussvorrichtung angeschlossenen Instruments zu folgen, und eine magnetische Kupplungsvorrichtung, die zum Bewegen des Rahmens relativ zur zumindest einen Anschlussvorrichtung eine außerhalb des Reinigungs- oder Pflegeraums erzeugte Antriebsbewegung auf den Rahmen überträgt.

Durch das Vorsehen der magnetischen Kupplungsvorrichtung sind zum Bewegen des Rahmens keine weiteren Bauteile innerhalb des Reinigungs- oder Pflegeraums notwendig. Damit steht in vorteilhafter Weise der Reinigungs- oder Pflegeraum ausschließlich für die Aufnahme der zu reinigenden Instrumente zur Verfügung, es geht kein Raum für die Aufnahme der zu reinigenden Instrumente verloren und die Reinigung des Reinigungs- oder Pflegeraums wird erleichtert.

Als Reinigungs- oder Pflegegerät im Sinne des vorliegenden Schriftsatzes wird jede Vorrichtung bezeichnet, die zumindest ein Reinigungs- und/oder Pflegemittel an ein medizinisches, insbesondere dentales, Instrument abgibt, um dieses zu reinigen, zu desinfizieren, zu sterilisieren, von Verschmutzungen oder Mikroorganismen zu befreien oder zu pflegen. Als Reinigungs- oder Pflegemittel können zum Beispiel eine Flüssigkeit, insbesondere Heiß- oder Kaltwasser, Dampf, Desinfektions- oder Sterilisationsmittel, ein Gas, zum Beispiel Druckluft, oder ein Schmiermittel, zum Beispiel natürliche oder synthetische Schmieröle, verwendet werden.

Als zu reinigende medizinische, insbesondere dentale, Instrumente werden insbesondere Hand- und Winkelstücke zum Antrieb verschiedenster Werkzeuge, wie rotierender Bohrer, Zahnsteinentfernungswerkzeuge, Feilen, Sägen, Reamer usw., Handstücke zur Abgabe von medizinischen Materialien, wie zum Beispiel Füllmittel oder Anästhetika, Funktionshandstücke zur Abgabe von Licht, Wasser oder Luft und nicht angetriebene Handinstrumente, wie zum Beispiel Endoskope und dergleichen, verstanden.

Die Fördervorrichtung zur Förderung zumindest eines Reinigungs- oder Pflegemediums umfasst neben dem Rahmen weitere Bauteile, zum Beispiel eine oder mehrere Leitungen oder Kanäle für ein Reinigungs- oder Pflegemedium, eine oder mehrere Pumpen zum Fördern eines Reinigungs- oder Pflegemediums, ein oder mehrere Ventile, Drosseln, Durchflussmessvorrichtungen, etc. Das oder die Reinigungs- oder Pflegemedien sind entweder in Behältern oder Aufnahmen in dem Reinigungs- oder Pflegegerät gelagert und / oder sie werden dem Reinigungs- oder Pflegegerät über Anschlüsse am Reinigungs- oder Pflegegerät von externen Quellen zugeführt.

Der Rahmen ist relativ zur zumindest einen Anschlussvorrichtung bewegbar, um im Wesentlichen der Längsachse oder, im Falle eines gewinkelten Instruments, einer Längsachse eines an die zumindest eine Anschlussvorrichtung angeschlossenen Instruments zu folgen. Der Rahmen bewegt sich somit im Reinigungs- oder Pflegeraum längs oder entlang (der Länge) des zu reinigenden oder zu pflegenden Instruments, insbesondere entlang des gesamten Instruments oder entlang der gesamten Länge des Instruments, wodurch sicher gestellt wird, dass das gesamte Instrument mit dem Reinigungs-oder Pflegemedium beaufschlagt wird, insbesondere im Wesentlichen gleichmäßig über seine gesamte Länge. Dadurch ist eine besonders gründliche Reinigung oder Pflege erzielbar.

Zusätzlich oder alternativ bewegt sich der Rahmen im Reinigungs- oder Pflegeraum entlang der Längsachse der zumindest einen Anschlussvorrichtung oder entlang der Verlängerung der Längsachse der zumindest einen Anschlussvorrichtung (Verlängerung in den Reinigungs- oder Pflegeraum), wodurch ebenfalls eine besonders gründliche Reinigung oder Pflege erzielbar ist.

Gemäß einem Ausführungsbeispiel weist die magnetische Kupplungsvorrichtung zumindest ein außerhalb des Reinigungs- oder Pflegeraums angeordnetes, mit einer Antriebsvorrichtung verbundenes, erstes Magnetelement auf, das mit zumindest einem mit dem Rahmen verbundenen, zweiten Magnetelement magnetisch gekoppelt ist. In vorteilhafter Weise ist damit auch der Antrieb für den Rahmen bzw. für die Bewegung des Rahmens außerhalb des Reinigungs- oder Pflegeraums angeordnet. Bevorzugt ist auch das erste Magnetelement außerhalb des Reinigungs- oder Pflegeraums angeordnet, so dass das erste Magnetelement und das zweite Magnetelement durch eine Wand des Reinigungs- oder Pflegeraums voneinander getrennt sind. Besonders bevorzugt sind die beiden Magnetelemente jeweils unmittelbar oder angrenzend an der Wand des Reinigungs- oder Pflegeraums angeordnet, wodurch eine besonders starke magnetische Kopplung erzielbar ist.

Um eine gleichmäßige Bewegung des Rahmens entlang seines gesamten Bewegungsweges zu erzielen, umfasst gemäß einem Ausführungsbeispiel die mit dem ersten Magnetelement verbundene Antriebsvorrichtung einen Linearantrieb, der das erste Magnetelement entlang einer Wand des Reinigungs- oder Pflegeraums bewegt. Bevorzugt ist die mit dem ersten Magnetelement verbundene Antriebsvorrichtung als Riemenantrieb ausgebildet.

Um die Bewegung des Rahmens zu stabilisieren oder zu zentrieren, ist gemäß einem Ausführungsbeispiel am Rahmen und / oder an einer Wand des Reinigungs- oder Pflegeraums eine Leitvorrichtung zum Leiten des Rahmens entlang der Wand des Reinigungs- oder Pflegeraums vorgesehen. Die Leitvorrichtung ist zum Beispiel als geometrische Form ausgebildet, insbesondere als geometrische Form mit geometrisch komplementären Elementen am Rahmen und an der Wand.

Um eine leichtgängige Bewegung an oder entlang der Wand des Reinigungs- oder Pflegeraums zu erreichen, weist der Rahmen gemäß einem Ausführungsbeispiel einen oder mehrere vom Körper des Rahmens abstehende Fortsätze auf, mit denen der Rahmen an einer Wand des Reinigungs- oder Pflegeraums gleitet.

Gemäß einem weiteren Ausführungsbeispiel weist der Körper des Rahmens eine Durchbohrung auf, in welcher ein zu reinigendes Instrument aufnehmbar oder durch welche ein zu reinigendes Instrument durchführbar ist, wobei an der geschlossenen Innenwand der Durchbohrung mehrere Düsen angeordnet sind. Damit wird sichergestellt, dass das zu reinigende Instrument gründlich, gleichmäßig und von allen Seiten mit dem Reinigungs- oder Pflegemedium beaufschlagt wird.

Gemäß einem anderen Ausführungsbeispiel weist das Reinigungs- oder Pflegegerät eine Detektionsvorrichtung zum Nachweis einer Relativbewegung zwischen dem Rahmen und dem ersten Magnetelement auf. Bevorzugt umfasst die Detektionsvorrichtung einen Magnetsensor, der gemeinsam mit dem außerhalb des Reinigungs- oder Pflegeraums angeordneten, ersten Magnetelement bewegbar ist und der operativ mit einem am Rahmen vorgesehenen Magnetelement verbunden ist. Bei dem am Rahmen vorgesehenen Magnetelement kann es sich entweder um das zweite Magnetelement handeln oder um ein weiteres, drittes Magnetelement. Der Magnetsensor, zum Beispiel ein Reed- oder Hallsensor, ist ausgebildet, das am Rahmen vorgesehene Magnetelement zu detektieren, zum Beispiel über dessen Magnetfeld. Eine Relativbewegung zwischen dem Rahmen und dem ersten Magnetelement tritt zum Beispiel auf, wenn der Rahmen nicht durch das erste Magnetelement oder nicht gemeinsam mit dem ersten Magnetelement bewegt wird. In diesem Fall entfernt sich der Magnetsensor vom am Rahmen vorgesehenen Magnetelement bis der Magnetsensor das am Rahmen vorgesehenen Magnetelement nicht (mehr) detektiert. In diesem Fall sendet der Magnetsensor ein Signal an eine Steuervorrichtung, die den Anwender über die Relativbewegung zwischen dem Rahmen und dem ersten Magnetelement informiert, zum Beispiel mittels einer optischen oder akustischen Warnvorrichtung, und / oder die den Betrieb des Reinigungs- oder Pflegegeräts unterbricht oder unterbindet.

Um auch das Innere eines an die zumindest eine Anschlussvorrichtung angeschlossenen Instruments reinigen zu können, weist gemäß einem Ausführungsbeispiel die zumindest eine Anschlussvorrichtung einen mit der Fördervorrichtung zur Förderung zumindest eines Reinigungs- oder Pflegemediums verbundenen Kanal auf, über den ein Reinigungs- oder Pflegemedium in das Innere eines Instruments förderbar ist.

Gemäß einem bevorzugten Ausführungsbeispiel sind zumindest zwei Anschlussvorrichtungen für zu reinigende Instrumente vorgesehen, die derart relativ zu dem Rahmen bewegbar sind, dass jeweils nur eine der Anschlussvorrichtungen einer Durchbohrung des Rahmens gegenüber steht, an welcher die zumindest eine Düse zur Abgabe eines Reinigungs- oder Pflegemediums vorgesehen ist. Besonders bevorzugt sind die zumindest zwei Anschlussvorrichtungen auf einem gemeinsamen Basiselement angeordnet, das drehbar im Reinigungs- oder Pflegegerät angeordnet ist. Durch diese Ausführungsbeispiele ist ein besonders kompaktes Reinigungs- oder Pflegegerät geschaffen, bei dem zumindest die Außenreinigung oder -pflege, bevorzugt zusätzlich auch die Innenreinigung oder -pflege, der Instrumente sequentiell erfolgt: Die Durchbohrung mit der zumindest eine Düse ist derart bemessen, dass jeweils nur ein Instrument darin aufnehmbar ist, so dass, wenn der Rahmen sich entlang dieses Instruments bewegt, zur Außenreinigung oder -pflege jeweils nur dieses eine Instrument von der zumindest einen Düse mit einem Reinigungs- oder Pflegemedium beaufschlagbar ist.

Ein Verfahren zur Herstellung eines Reinigungs- oder Pflegegeräts ist dadurch definiert, dass das Reinigungs- oder Pflegegerät mit einer magnetische Kupplungsvorrichtung versehen wird, die zum Bewegen des Rahmens relativ zur zumindest einen Anschlussvorrichtung eine außerhalb des Reinigungs- oder Pflegeraums erzeugte Antriebsbewegung auf den Rahmen überträgt. Bevorzugt wird das Reinigungs- oder Pflegegerät mit zumindest zwei Anschlussvorrichtungen für zu reinigende Instrumente versehen, die derart relativ zu dem Rahmen bewegbar sind, dass jeweils nur eine der Anschlussvorrichtungen einer Durchbohrung des Rahmens gegenüber steht, an welcher die zumindest eine Düse zur Abgabe eines Reinigungs- oder Pflegemediums vorgesehen ist.

Die Erfindung wird nachfolgend anhand bevorzugter Ausführungsbeispiele und Bezug nehmend auf die beigefügten Zeichnungen erläutert:
Figur 1 zeigt ein Ausführungsbeispiel eines Reinigungs- oder Pflegegeräts mit einem durch eine magnetische Kupplungsvorrichtung bewegten Rahmen.
Figur 2A zeigt eine Schnittdarstellung durch das Reinigungs- oder Pflegegerät der Figur 1 ohne an die Anschlussvorrichtungen angeschlossene Instrumente.
Figur 2B zeigt die gleiche Schnittdarstellung wie die Figur 2A, jedoch mit einem an eine Anschlussvorrichtung angeschlossenen Instrument.
Figur 3 zeigt eine Außenansicht einer Ausführungsform eines durch eine magnetische Kupplungsvorrichtung bewegbaren Rahmens.
Figur 4 zeigt einen Längsschnitt durch den Rahmen der Figur 3.
Figur 5 zeigt einen Querschnitt durch den Rahmen der Figur 3.

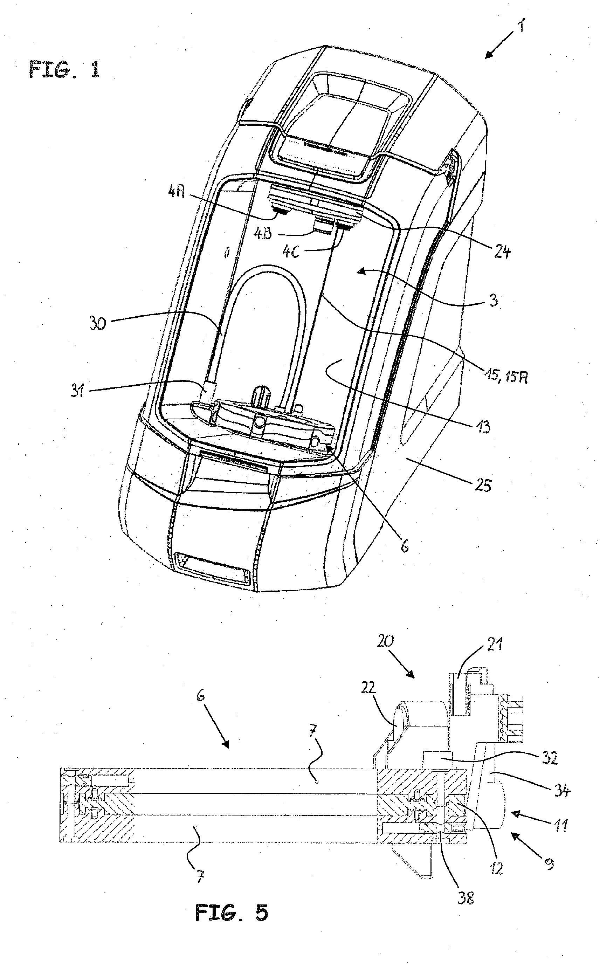

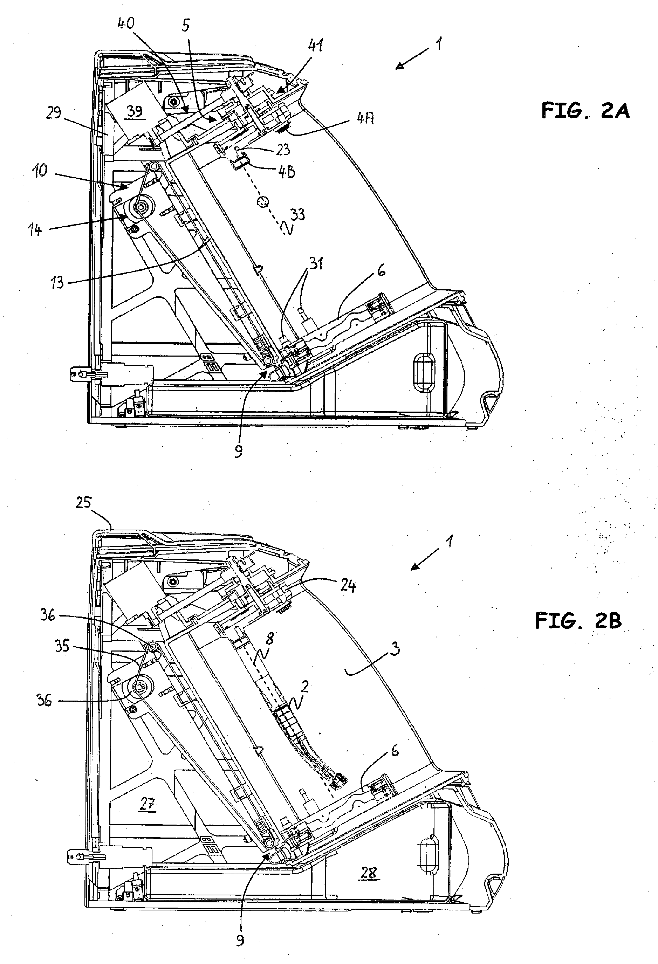

Das in den Figuren 1, 2A und 2B dargestellte Reinigungs- oder Pflegegerät 1 umfasst ein, bevorzugt aus Kunststoff hergestelltes, Außengehäuse 25 mit einer Bodenplatte, mehreren Seitenwänden und einer Oberplatte. Eine der Seitenwände weist eine bewegliche, insbesondere verschwenkbare, Türe oder einen beweglichen, insbesondere verschwenkbaren, Deckel auf, der bevorzugt über Scharniere an der Oberplatte bewegbar ist. Mit dem Deckel ist eine im Inneren des Gehäuses 25 angeordnete Reinigungs- oder Pflegekammer oder ein im Inneren des Gehäuses 25 angeordneter Reinigungs- oder Pflegeraum 3 verschließbar, in dem mehrere Kupplungen oder Anschlussvorrichtungen, im vorliegenden Fall drei Anschlussvorrichtungen 4A, 4B, 4C, für zu reinigende oder zu pflegende medizinische, insbesondere dentale, Instrumente 2 vorgesehen sind. Mittels der Anschlussvorrichtungen 4A, 4B, 4C und eines oder mehrerer darin vorgesehener Kanäle 23 werden ein oder mehrere Reinigungs- oder Pflegemedien in das Innere der Instrumente 2 gefördert. Zusätzlich befindet sich in dem Reinigungs- oder Pflegeraum 3 ein Rahmen 6 mit zumindest einer Düse 7, über die ein oder mehrere Reinigungs- oder Pflegemedien auf die Außenseite der Instrumente 2 abgegeben werden können. Die zumindest eine Düse 7 ist somit von den Anschlussvorrichtungen 4A, 4B, 4C getrennt angeordnet und, insbesondere zeitlich, unabhängig von den Anschlussvorrichtungen 4A, 4B, 4C mit einem Reinigungs- und/oder Pflegemedium versorgbar.

Der Reinigungs- oder Pflegeraum 3 wird durch eine im Inneren des Reinigungs- oder Pflegegeräts 1 angeordnete Wand 13 von einem Steuerraum 27 (siehe Figur 2B) getrennt. Am oder im Steuerraum 27 sind eine Vielzahl von Steuer- und Funktionselementen des Reinigungs- oder Pflegegeräts 1 vorgesehen, zum Beispiel eine Versorgungs- oder Fördervorrichtung 5, die unter anderem Anschlüsse an eine oder mehrere Fluidquellen, zum Beispiel an Behälter für Reinigungs-, Pflege- oder Schmiermittel oder an eine externe Druckluft- und oder Wasserquelle, Leitungen zur Förderung der Fluide, Reinigungs-, Pflege- oder Schmiermittel, Pumpen, Steuer- und Stellmittel, zum Beispiel Ventile, Drosseln, Sensoren, zum Beispiel zur Durchflussmessung, Konzentrationsmessung, Mischer und viele weitere Bauteile enthalten kann. Neben der Fördervorrichtung 5 können am oder im Steuerraum 27 weitere Bauteile, wie zum Beispiel Behälter für Reinigungs-, Pflege- oder Schmiermittel oder Aufnahmen für derartige Behälter, ein Anschluss an eine Energiequelle, eine Auffangwanne 28 oder ein Ablauf für gebrauchte Reinigungs- oder Pflegemittel, eine Steuervorrichtung 29 zur Steuerung und / oder Regelung der Reinigungs- oder Pflegeverfahren, eine Speichereinheit, eine Betriebsanzeige oder ein Bedienpanel für den Anwender vorgesehen sein.

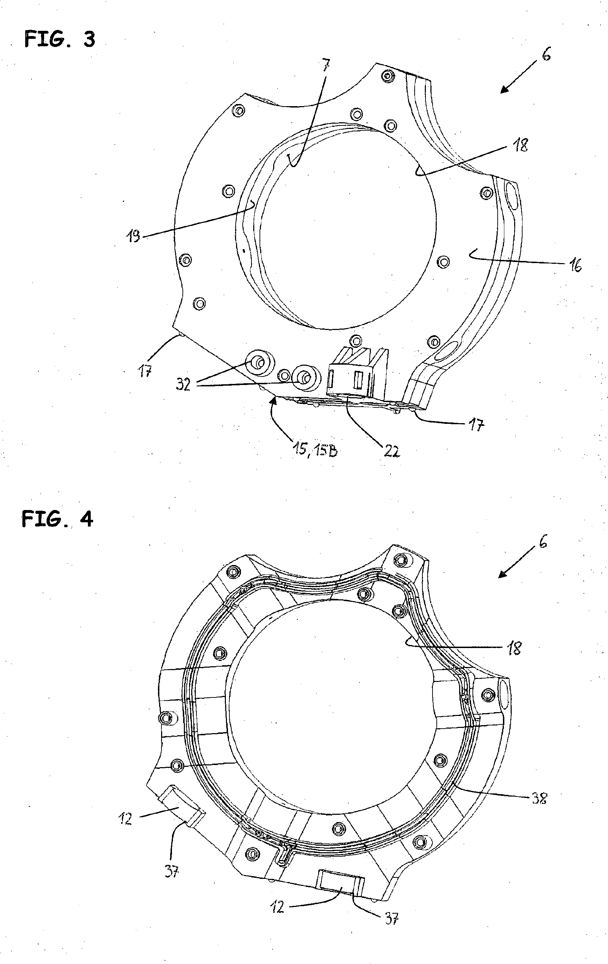

Im Reinigungs- oder Pflegeraum 3 sind die Anschlussvorrichtungen 4A, 4B, 4C oder zumindest Teile davon, der Rahmen 6 und zumindest ein flexibler, mit dem Rahmen 6 verbundener Leitungsschlauch 30 (siehe Figur 1) aufgenommen. Der flexible Leitungsschlauch 30 ist Teil der Fördervorrichtung 5 oder mit dieser verbunden und leitet ein oder mehrere Reinigungs- oder Pflegemedien zum Rahmen 6. An einem seiner beiden Enden ist der Leitungsschlauch 30 mit einem ersten Anschlusselement 31 verbunden, das an einer Wand des Reinigungs- oder Pflegeraums 3 vorgesehen ist und das ein Reinigungs- oder Pflegemedium von im Steuerraum 27 angeordneten Leitungen übernimmt und an den Leitungsschlauch 30 weiterleitet. An seinem anderen, zweiten Enden ist der Leitungsschlauch 30 mit einem zweiten Anschlusselement 32 verbunden, das am Rahmen 6 angeordnet ist und das das Reinigungs- oder Pflegemedium vom Leitungsschlauch 30 übernimmt und an den Rahmen 6 überträgt. Die beiden Anschlusselemente 31, 32 sind zum Beispiel als Rohrstutzen oder Buchsen ausgebildet und durch Steckverbindungen mit dem Leitungsschlauch 30 lösbar verbunden. Wie aus den Figuren 2A, 2B und 3 zu erkennen ist, ist es selbstverständlich möglich, mehrere Anschlusselemente 31, 32 vorzusehen, wobei jeweils ein Anschlusselement 31 über einen Leitungsschlauch 30 mit einem Anschlusselement 32 verbunden ist, so dass mehrere voneinander getrennte Versorgungswege für Reinigungs- oder Pflegemedien zum Rahmen 6 gebildet werden.

Um die Instrumente 2 besonders gründlich reinigen oder pflegen zu können, ist der Rahmen 6 beweglich in dem Reinigungs- oder Pflegeraum 3 aufgenommen. Der Rahmen 6 ist ausgebildet, sich entlang einer Wand 13 des Reinigungs- oder Pflegeraums 3 und / oder relativ zu den Anschlussvorrichtung 4A, 4B, 4C und / oder entlang einer Längsachse 8 eines an eine Anschlussvorrichtung 4A, 4B, 4C angeschlossenen Instruments 2 und / oder entlang der Längsachse 33 einer Anschlussvorrichtung 4A, 4B, 4C und / oder entlang der Verlängerung der Längsachse 33 (in den Reinigungs- oder Pflegeraum 3) der zumindest einen Anschlussvorrichtung 4A, 4B, 4C zu bewegen.

Die Bewegung des Rahmens 6 wird durch eine magnetische Kupplungsvorrichtung 9 bewirkt, die eine Antriebsbewegung einer im Steuerraum 27 angeordneten Antriebsvorrichtung 10 auf den Rahmen 6 überträgt. Die magnetische Kupplungsvorrichtung 9 besteht aus zwei Teilen, wobei ein mit der Antriebsvorrichtung 10 verbundener Teil im Steuerraum 27 angeordnet ist und ein zweiter Teil mit dem Rahmen 6 verbunden ist. Die beiden Teile der magnetischen Kupplungsvorrichtung 9 sind durch eine Wand 13 des Reinigungs- oder Pflegeraums 3 räumlich voneinander getrennt, aber derart im Reinigungs- oder Pflegegerät 1 angeordnet, dass sie magnetisch miteinander gekoppelt sind.

Der im Steuerraum 27 angeordnete Teil der magnetischen Kupplungsvorrichtung 9 umfasst zumindest ein, bevorzugt mehrere, erste Magnetelemente 11, die in oder an einer Magnethaltevorrichtung 34 befestigt sind. Das erste Magnetelement 11 ist bevorzugt nahe der Wand 13 oder unmittelbar anschließend an die Wand 13 angeordnet. Die Magnethaltevorrichtung 34 ist zum Beispiel aus Kunststoff hergestellt und umschließt das erste Magnetelement 11 zumindest teilweise. Bevorzugt dient die Magnethaltevorrichtung 34 auch als Verbindungsstück zur Antriebsvorrichtung 10, wobei selbstverständlich auch ein separates Verbindungsstück dafür vorgesehen sein kann.

Die Antriebsvorrichtung 10 für das erste Magnetelement 11 ist mit einem Motor verbunden, zum Beispiel einen Elektromotor, insbesondere einem Schrittmotor, dessen Antriebsbewegung auf das erste Magnetelement 11 übertragen wird. Die Übertragung der Antriebsbewegung erfolgt gemäß den Ausführungsformen der Figuren 2A, 2B mittels eines Riemenantriebs 14, sie kann jedoch auch durch beliebige andere bekannte Mittel bewerkstelligt werden, zum Beispiel durch Drehung einer Gewindestange, an der das das erste Magnetelement 11 befestigt ist.

Der Riemenantrieb 14 umfasst einen Riemen 35, insbesondere einen Zahnriemen, an dem das erste Magnetelement 11 befestigt ist, zum Beispiel über die Magnethaltevorrichtung 34. Der Riemen 35 läuft über zwei oder mehr Riemenscheiben 36, wobei eine Riemenscheibe 36 über eine Welle mit dem Motor verbunden ist und durch diese in Drehung versetzt wird. Jener Teil des Riemens 35, an dem das erste Magnetelement 11 befestigt ist, erstreckt sich entlang der Wand 13 des Reinigungs- oder Pflegeraums 3, insbesondere im Wesentlichen parallel zur Wand 13. Sobald der Motor aktiviert wird, wird somit das erste Magnetelement 11 entlang der Wand 13 bewegt. Die Steuervorrichtung 29 steuert den Motor derart, dass dieser die Drehrichtung umkehrt, oder die Steuervorrichtung 29 stoppt den Motor, sobald das erste Magnetelement 11 einen Endpunkt seiner Wegstrecke entlang der Wand 13 erreicht. Ein Endpunkt kann zum Beispiel durch eine Riemenscheibe 36 definiert sein oder durch einen beliebigen anderen Punkt, über den hinaus das erste Magnetelement 11 oder das damit magnetisch gekoppelte zweite Magnetelement 12 des Rahmens oder der Rahmen 6 nicht bewegt werden sollen.

Der zweite, mit dem Rahmen 6 verbundene Teil der magnetischen Kupplungsvorrichtung 9 umfasst zumindest ein, bevorzugt mehrere, zweite Magnetelemente 12, die in Aufnahmen oder Rücksprüngen 37 des Rahmens 6 angeordnet sind (siehe Figur 4). Die zweiten Magnetelemente 12 sind bevorzugt nahe jener Wand 13 oder unmittelbar anschließend an jene Wand 13 angeordnet, an der (an der gegenüberliegenden Seite) auch das erste Magnetelement 11 positioniert ist. Die zweiten Magnetelemente 12 sind insbesondere an einer Seite oder Fläche des Rahmens 6 angeordnet, die jener Wand 13 des Reinigungs- oder Pflegeraums 3 zugewandt ist, an der (an der gegenüberliegenden Seite) auch das erste Magnetelement 11 positioniert ist. Damit wird eine besonders gute magnetische Kopplung der Magnetelemente 11, 12 erzielt. Bewegt sich, wie weiter oben bereits beschrieben, das erste Magnetelement 11 entlang der Wand 13, so folgt aufgrund der magnetischen Kopplung der Magnetelemente 11, 12 das zweite Magnetelement 12 und damit der gesamte Rahmen 6 dieser Bewegung des ersten Magnetelements 11. Der Rahmen 6 kontaktiert dabei zumindest teilweise die Wand 13.

Um eine leichtgängige Bewegung an oder entlang der Wand 13 des Reinigungs- oder Pflegeraums 3 zu erreichen, weist der Rahmen 6 mehrere Fortsätze 17 auf, die zum Beispiel als Nasen oder halbkugelige oder ballige Vorsprünge ausgebildet sind. Die Fortsätze 17 sind bevorzugt an jener Seite oder Fläche des Rahmens 6 angeordnet, an welcher die zweiten Magnetelemente 12 oder deren Aufnahmen 37 vorgesehen sind. Die Fortsätze 17 bewirken, dass nicht die gesamte Seite oder Fläche des Rahmens 6, an welcher die zweiten Magnetelemente 12 angeordnet sind, an der Wand 13 gleitet.

Zum Zentrieren und Führen des Rahmens 6 entlang der Wand 13 ist am Rahmen 6, insbesondere an jener Seite oder Fläche des Rahmens 6, an welcher die zweiten Magnetelemente 12 oder deren Aufnahmen 37 vorgesehen sind, und an der Wand 13 eine Leitvorrichtung 15 vorgesehen. Die Leitvorrichtung 15 weist korrespondierende geometrische Formen am Rahmen 6 und an der Wand 13 auf, die zum Beispiel als gewinkelt zueinander angeordnete Abschnitte einer Seitenwand des Rahmens 6 und der Wand 13 ausgebildet sind und die im Kontaktbereich eine Spitze oder Kante 15B (siehe Figur 3) und einen korrespondierenden Winkel 15A bilden (siehe Figur 1). Bewegt sich der Rahmen 6 entlang der Wand 13, so wird die Kante 15B in dem Winkel 15A geführt. Selbstverständlich kann die Leitvorrichtung 15 auch andere geometrische Formen aufweisen, zum Beispiel Leisten, die in entsprechenden Rücksprüngen aufgenommen und geführt sind.

Wie insbesondere aus der Figur 5 zu erkennen ist, ist am Rahmen 6 ein drittes Magnetelement 22 vorgesehen, das Teil einer Detektionsvorrichtung 20 zum Nachweis einer Relativbewegung zwischen dem Rahmen 6 und dem ersten Magnetelement 11 ist. Eine derartige Relativbewegung tritt zum Beispiel auf, wenn der Rahmen 6 nicht durch das erste Magnetelement 11 oder nicht gemeinsam mit dem ersten Magnetelement 11 bewegt wird. Die Detektionsvorrichtung 20 umfasst des Weiteren einen Magnetsensor 21, der gemeinsam mit dem außerhalb des Reinigungs- oder Pflegeraums 3 angeordneten, ersten Magnetelement 11 bewegbar ist und der operativ mit dem am Rahmen 6 vorgesehenen Magnetelement 22 verbunden ist. Bevorzugt ist der Magnetsensor 21, zum Beispiel ein Hall- oder ein Reed-Sensor, an der Magnethaltevorrichtung 34 befestigt. Der Magnetsensor 21 detektiert das Magnetfeld des dritten Magnetelements 22. Entfernt sich der Magnetsensor 21 vom dritten Magnetelement 22, so detektiert der Magnetsensor 21 dessen Magnetfeld nicht mehr oder der Magnetsensor 21 detektiert die Abnahme der magnetischen Feldstärke. In beiden Fällen sendet der Magnetsensor 21 ein Signal an die Steuervorrichtung 29, die den Anwender über die Relativbewegung zwischen dem Rahmen 6 und dem ersten Magnetelement 11 informiert, zum Beispiel mittels einer optischen oder akustischen Warnvorrichtung, und / oder die den Betrieb des Reinigungs- oder Pflegegeräts unterbricht oder unterbindet. Sowohl der Magnetsensor 21 als auch das dritte Magnetelement 22 sind nahe zueinander angeordnet, insbesondere nahe der Wand 13. Das dritte Magnetelement 22 ist bevorzugt an oder nahe jener Seite oder Fläche des Rahmens 6 angeordnet, an welcher die zweiten Magnetelemente 12 oder deren Aufnahmen 37 vorgesehen sind. Gemäß einem alternativen Ausführungsbeispiel detektiert der Magnetsensor 21 das zweite Magnetelement 12, so dass kein drittes Magnetelement 22 benötigt wird.

Der Körper 16 des Rahmens 6 ist von einer oder mehreren Kanälen oder Leitungen 38 durchsetzt, die mit zumindest jeweils einem Anschlusselement 32 verbunden sind, um ein Reinigungs- oder Pflegemedium von dem Anschlusselement 32 zu den Düsen 7 zu leiten. Die Düsen 7 bzw. die Düsenöffnungen sind an einer Innenwand 19 einer den Körper 16 durchsetzenden Durchbohrung 18 vorgesehen. Die Durchbohrung 18 ist als geschlossene, insbesondere kreisrunde, Bohrung ausgebildet, deren Durchmesser bevorzugt derart bemessen ist, dass jeweils nur ein einziges zu reinigendes Instrument 2 darin Platz findet. Demgemäß sind die Düsen 7 während des Reinigungs- oder Pflegeverfahrens immer nur auf ein Instrument 2 gerichtet und geben nur auf dieses Instrument 2 (direkt) ein Reinigungs- oder Pflegemedium ab.

Der Körper 16 des Rahmens 6 besteht bevorzugt aus mehreren Schichten oder Lagen, in denen oder an deren aneinandergrenzenden Flächen die Leitungen 38 vorgesehen sind. Gemäß der in der Figur 5 dargestellten Ausführungsform besteht der Rahmen 6 aus drei Schichten, wobei die beiden äußeren Schichten aus Kunststoff bestehen und die mittlere Schicht als Dichtelement ausgebildet ist und aus einem Material mit einer höheren Elastizität als die beiden Außenschichten besteht, zum Beispiel aus Gummi.

Aus den Figuren 1, 2A, 2B ist schließlich noch zu erkennen, dass die drei Anschlussvorrichtungen 4A, 4B, 4C auf einem gemeinsamen Basiselement 24 angeordnet sind, das drehbar in dem Reinigungs- oder Pflegegerät 1 angeordnet ist. Aufgrund der Drehbarkeit des Basiselements 24 sind die drei Anschlussvorrichtungen 4A, 4B, 4C relativ zum Rahmen 6 bewegbar, d.h. drehbar. Insbesondere sind die drei Anschlussvorrichtungen 4A, 4B, 4C derart relativ zum Rahmen 6 drehbar und / oder sind die drei Anschlussvorrichtungen 4A, 4B, 4C derart in einer vorbestimmten Stoppposition anordenbar und / oder sind der Rahmen 6 und das Basiselement 24 durch Drehen des Basiselements 24 derart relativ zueinander anordenbar, dass jeweils nur ein zu reinigendes Instrument 2 und / oder jeweils nur eine Anschlussvorrichtungen 4A, 4B, 4C (direkt) gegenüber der Durchbohrung 18 des Rahmens 6 angeordnet ist. Ist der Durchmesser der Durchbohrung 18 derart bemessen, dass jeweils nur ein einziges zu reinigendes Instrument 2 darin aufnehmbar ist, so wird, sobald sich der Rahmen 6 entlang einer Längsachse 8 des Instruments 2 bewegt, nur dieses in der Durchbohrung 18 aufgenommene Instrument 2 (direkt) von den Düsen 7 mit einem Reinigungs- oder Pflegemedium beaufschlagt. Die Außenreinigung oder -pflege der Instrumente 2 erfolgt somit sequentiell.

Ein Reinigungs- oder Pflegegerät mit einem drehbaren Basiselement, auf dem mehrere Anschlussvorrichtungen angeordnet sind, ist auch aus der Patentanmeldung EP 2364665 bekannt Das Reinigungs- oder Pflegegerät der Patentanmeldung EP 2364665 weist eine einzige Fördervorrichtung für die Reinigungs- oder Pflegemedien auf, mit der durch Drehen des Basisteils jeweils nur eine Anschlussvorrichtungen verbunden ist, während alle anderen Anschlussvorrichtungen nicht mit der Fördervorrichtung verbunden sind, so dass jeweils nur ein Instrument mit einem Reinigungs- oder Pflegemedium zur Innenreinigung versorgbar ist.

Dem entsprechend sind gemäß einem bevorzugten Ausführungsbeispiel auch bei dem gegenständlichen Reinigungs- oder Pflegegerät 1 das Basiselement 24 und damit die drei Anschlussvorrichtungen 4A, 4B, 4C und die Fördervorrichtung 5 derart relativ zueinander bewegbar, dass (wahlweise) nur eine der Anschlussvorrichtungen 4A, 4B, 4C mit der Fördervorrichtung 5 verbunden und mit einem Reinigungs- und/oder Pflegemedium für die Innenreinigung eines Instruments 2 versorgbar ist, während die anderen Anschlussvorrichtungen 4A, 4B, 4C nicht mit der Fördervorrichtung 5 verbunden sind und nicht mit einem Reinigungs- und/oder Pflegemedium versorgbar sind. Das Reinigungs- oder Pflegegerät 1 weist somit eine einzige Fördervorrichtung 5 auf, mit der jeweils nur eine Anschlussvorrichtungen 4A, 4B, 4C bzw. deren Kanal 23 verbunden ist, während alle anderen Anschlussvorrichtungen 4A, 4B, 4C nicht mit der Fördervorrichtung 5 verbunden sind, so dass jeweils nur ein Instrument 2 mit einem Reinigungs- oder Pflegemedium zur Innenreinigung versorgbar ist. Insbesondere umfasst das Reinigungs- oder Pflegegerät 1 oder die Fördervorrichtung 5 eine einzige Schnittstelle oder Übergabestelle zur wahlweisen Kopplung oder Verbindung mit einer der Anschlussvorrichtungen 4A, 4B, 4C, an der das oder die Reinigungs- oder Pflegemedien übergeben werden. An dieser Schnittstelle können klarerweise mehrere Leitungen für Reinigungs- oder Pflegemittel vorgesehen oder zusammengefasst sein. Bevorzugt weist die Schnittstelle zumindest eine Öffnung für ein Reinigungsfluid und zumindest eine Öffnung für ein Schmiermittel auf.

Bei einem derartigen Reinigungs- oder Pflegegerät 1 erfolgt somit sowohl die Auenreinigung oder -pflege der Instrumente 2 als auch die Innenreinigung oder -pflege der Instrumente 2 sequentiell, wobei gemäß einem Ausführungsbeispiel bei einem ersten Instrument 2 die Außen- und die Innenreinigung / -pflege durchgeführt wird und anschließend sich das Basiselement 24 dreht, so dass anschließend bei einem zweiten oder weiteren Instrument 2 die Außen- und die Innenreinigung / -pflege durchgeführt wird. Alternativ wird zuerst bei allen Instrumenten 2 eine der beiden Reinigungen / Pflegen (Innenreinigung oder -pflege / Außenreinigung oder -pflege) und anschließend die andere der beiden Reinigungen / Pflegen (Innenreinigung oder -pflege / Außenreinigung oder - pflege) vorgenommen. Bevorzugt wird zuerst bei allen Instrumenten 2 die Innenreinigung oder -pflege und anschließend bei allen Instrumenten 2 die Außenreinigung oder -pflege durchgeführt, wodurch die Gefahr einer Kreuzkontamination zwischen den Instrumenten 2 merklich reduziert wird. Besonders bevorzugt ist jene Position, in der ein zu reinigendes Instrument 2 der Durchbohrung 18 des Rahmens 6 (direkt) gegenüber angeordnet ist, gleich jener Position, in der die Anschlussvorrichtungen 4A, 4B, 4C, an welcher dieses der Durchbohrung 18 gegenüber liegende Instrument 2 angeschlossen ist, mit der Fördervorrichtung 5 verbunden ist.

Bevorzugt ist im Reinigungs- oder Pflegegerät 1 ein Antriebsmotor 39 zur Relativbewegung der Anschlussvorrichtungen 4A, 4B, 4C oder des Basiselements 24 vorgesehen. Der Antriebsmotor 39 ist außerhalb des Reinigungs- oder Pflegeraums 3 angeordnet. Er umfasst zum Beispiel einen Elektromotor, insbesondere einen Schrittmotor. Die Verwendung eines Schrittmotors ist aufgrund seiner Zuverlässigkeit und einfachen Steuerung für die vorliegende Anwendung von besonderem Vorteil. Bevorzugt ist der Antriebsmotor 39 über zumindest ein Antriebselement, zum Beispiel einen Riemenantrieb 40 und / oder eine Welle, mit den Anschlussvorrichtungen 4A, 4B, 4C und / oder dem Basiselement 24 verbunden.

Das Reinigungs- oder Pflegegerät 1 umfasst des Weiteren ein zwischen dem bewegbaren oder drehbaren Basiselement 24 und der Fördervorrichtung 5 vorgesehenes Verteilerelement 41 zum Leiten eines Reinigungs- und/oder Pflegemediums von der Fördervorrichtung 5 zu den Anschlussvorrichtungen 4A, 4B, 4C, wobei das Verteilerelement 41 ein erstes, mit dem Basiselement 24 relativ zur Fördervorrichtung 5 bewegbares Verteilerstück und ein zweites, relativ zur Fördervorrichtung 5 festes Verteilerstück aufweist. Dieser Aufbau gewährleistet eine besonders zuverlässige Übergabe der Reinigungs- und/oder Pflegemittel an das Basiselement 24. Das erste Verteilerstück weist bevorzugt zumindest jeweils eine eigene Leitung oder einen eigenen Leitungsabschnitt für jede der Anschlussvorrichtungen 4A, 4B, 4C auf. Das zweite Verteilerstück umfasst zumindest eine Leitung für ein Reinigungs- und/oder Pflegemedium, wobei durch das Bewegen des Basiselements 24 und somit des ersten Verteilerstücks relativ zum zweiten Verteilerstück die Leitungen des ersten Verteilerstücks mit der zumindest einen Leitung des zweiten Verteilerstücks verbindbar sind. Über diese fluidleitende Verbindung können somit das oder die Reinigungs- oder Pflegemedien für die Innenreinigung der Instrumente 2 von der Fördervorrichtung 5 auf die Anschlussvorrichtungen 4A, 4B, 4C und in das Innere der jeweils mit den Anschlussvorrichtungen 4A, 4B, 4C gekuppelten, zu reinigenden Instrumente 2 übertragen werden. Bevorzugt sind das erste und zweite Verteilerstück scheibenförmig geformt.

Bevorzugt umfasst das Reinigungs- oder Pflegegerät 1 eine Detektionseinheit zur Bestimmung welcher der Anschlussvorrichtungen 4A, 4B, 4C mit einem Instrument 2 belegt ist und / oder welche der Anschlussvorrichtungen 4A, 4B, 4C nicht mit einem Instrument 2 belegt ist und zur Abgabe entsprechender Belegungssignale für jede Anschlussvorrichtungen 4A, 4B, 4C. Die Detektionseinheit ist mit der Steuervorrichtung 29 verbunden, die auf Basis der von der Detektionseinheit abgegebenen Belegungssignale dazu ausgebildet ist, nur jene der Anschlussvorrichtungen 4A, 4B, 4C mit Reinigungs- oder Pflegemedien zu versorgen, an denen ein Instrument 2 angeschlossen ist. Die Detektionseinheit besteht zum Beispiel aus einer Strahlungsquelle, insbesondere einer Lichtquelle, die eine Strahlung in den Reinigungs- oder Pflegeraum 3 abgibt, und einem optischen Sensor, der eine Schwächung oder Unterbrechung der in den Reinigungs- oder Pflegeraum 3 abgegebenen Strahlung durch ein an einen Anschluss gekuppeltes Instrument 2 erkennt. Ist eine Anschlussvorrichtungen 4A, 4B, 4C leer, d.h. nicht mit einem Instrument 2 belegt, dann betreibt gemäß einem bevorzugten Ausführungsbeispiel die Steuervorrichtung 29 den Antriebsmotor 39 derart, dass dieser nicht stoppt, wenn die leere Anschlussvorrichtungen 4A, 4B, 4C mit der Fördervorrichtung 5 verbunden ist, sondern dass er weiterläuft, so dass das Basiselement 24 weiter bewegt wird, bis die nächste mit einem Instrument 2 belegte Anschlussvorrichtungen 4A, 4B, 4C mit der Fördervorrichtung 5 verbunden ist.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Reinigungs- oder Pflegegerät (1) für medizinische Instrumente (2) umfassend: Einen Reinigungs- oder Pflegeraum (3), zumindest eine im Reinigungs- oder Pflegeraum (3) angeordneten Anschlussvorrichtung (4A, 4B, 4C), an die ein zu reinigendes Instrument (2) anschließbar ist, und eine Fördervorrichtung (5) zur Förderung zumindest eines Reinigungs- oder Pflegemediums, wobei die Fördervorrichtung (5) einen Rahmen (6) aufweist, an dem zumindest eine Düse (7) zur Abgabe eines Reinigungs- oder Pflegemediums an die Außenseite des zu reinigenden Instruments (2) vorgesehen ist, wobei der Rahmen (6) relativ zur zumindest einen Anschlussvorrichtung (4A, 4B, 4C) bewegbar ist, um im Wesentlichen einer Längsachse (8) eines an die zumindest eine Anschlussvorrichtung (4A, 4B, 4C) angeschlossenen Instruments (2) zu folgen, **gekennzeichnet, durch**
eine magnetische Kupplungsvorrichtung (9), die zum Bewegen des Rahmens (6) relativ zur zumindest einen Anschlussvorrichtung (4A, 4B, 4C) eine außerhalb des Reinigungs- oder Pflegeraums (3) erzeugte Antriebsbewegung auf den Rahmen (6) überträgt.

2. Reinigungs- oder Pflegegerät (1) nach Anspruch 1, **dadurch gekennzeichnet, dass** die magnetische Kupplungsvorrichtung (9) zumindest ein außerhalb des Reinigungs-oder Pflegeraums (3) angeordnetes, mit einer Antriebsvorrichtung (10) verbundenes, erstes Magnetelement (11) aufweist, das mit zumindest einem mit dem Rahmen (6) verbundenen, zweiten Magnetelement (12) magnetisch gekoppelt ist.

3. Reinigungs- oder Pflegegerät (1) nach Anspruch 2, **dadurch gekennzeichnet, dass** das erste Magnetelement (11) und das zweite Magnetelement (12) durch eine Wand (13) des Reinigungs- oder Pflegeraums (3) voneinander getrennt sind.

4. Reinigungs- oder Pflegegerät (1) nach Anspruch 2 oder 3, **dadurch gekennzeichnet, dass**
die mit dem ersten Magnetelement (11) verbundene Antriebsvorrichtung (10) einen Linearantrieb umfasst, der das erste Magnetelement (11) entlang einer Wand (13) des Reinigungs- oder Pflegeraums (3) bewegt.

5. Reinigungs- oder Pflegegerät (1) nach einem der Ansprüche 2 - 4, **dadurch gekennzeichnet, dass**
die mit dem ersten Magnetelement (11) verbundene Antriebsvorrichtung (10) einen Riemenantrieb (14) aufweist.

6. Reinigungs- oder Pflegegerät (1) nach einem der vorherstehenden Ansprüche, **dadurch gekennzeichnet, dass**
am Rahmen (6) und / oder an einer Wand (13) des Reinigungs- oder Pflegeraums (3) eine Leitvorrichtung (15, 15A, 15B) zum Leiten des Rahmens (6) entlang der Wand (13) des Reinigungs- oder Pflegeraums (3) vorgesehen ist.

7. Reinigungs- oder Pflegegerät (1) nach einem der vorherstehenden Ansprüche, **dadurch gekennzeichnet, dass**
der Rahmen (6) einen oder mehrere vom Körper (16) des Rahmens (6) abstehende Fortsätze (17) aufweist, mit denen der Rahmen (6) an einer Wand (13) des Reinigungs- oder Pflegeraums (3) gleitet.

8. Reinigungs- oder Pflegegerät (1) nach einem der vorherstehenden Ansprüche, **dadurch gekennzeichnet, dass**
der Körper (16) des Rahmens (6) eine Durchbohrung (18) aufweist, in welcher ein zu reinigendes Instrument (2) aufnehmbar ist, wobei an der geschlossenen Innenwand (19) der Durchbohrung (18) mehrere Düsen (7) angeordnet sind.

9. Reinigungs- oder Pflegegerät (1) nach einem der Ansprüche 2 - 8, **gekennzeichnet durch**
eine Detektionsvorrichtung (20) zum Nachweis einer Relativbewegung zwischen dem Rahmen (6) und dem ersten Magnetelement (11).

10. Reinigungs- oder Pflegegerät (1) nach Anspruch 9, **dadurch gekennzeichnet, dass** die Detektionsvorrichtung (20) einen Magnetsensor (21) umfasst, der gemeinsam mit dem außerhalb des Reinigungs- oder Pflegeraums (3) angeordneten, ersten Magnetelement (11) bewegbar ist und der operativ mit einem am Rahmen (6) vorgesehenen Magnetelement (12, 22) verbunden ist.

11. Reinigungs- oder Pflegegerät (1) nach einem der vorherstehenden Ansprüche, **dadurch gekennzeichnet, dass**
die zumindest eine Anschlussvorrichtung (4A, 4B, 4C) einen mit der Fördervorrichtung (5) zur Förderung zumindest eines Reinigungs- oder Pflegemediums verbundenen Kanal (23) aufweist, über den ein Reinigungs- oder Pflegemedium in das Innere eines Instruments (2) förderbar ist.

12. Reinigungs- oder Pflegegerät (1) nach einem der vorherstehenden Ansprüche, **dadurch gekennzeichnet, dass**
zumindest zwei Anschlussvorrichtungen (4A, 4B, 4C) für zu reinigende Instrumente (2) vorgesehen sind, die derart relativ zu dem Rahmen (6) bewegbar sind, dass jeweils nur eine der Anschlussvorrichtungen (4A, 4B, 4C) einer Durchbohrung (18) des Rahmens (6) gegenüber steht, an welcher die zumindest eine Düse (7) zur Abgabe eines Reinigungs- oder Pflegemediums vorgesehen ist.

13. Reinigungs- oder Pflegegerät (1) nach Anspruch 12, **dadurch gekennzeichnet, dass** die zumindest zwei Anschlussvorrichtungen (4A, 4B, 4C) auf einem gemeinsamen Basiselement (24) angeordnet sind, das drehbar im Reinigungs- oder Pflegegerät (1) angeordnet ist.

14. Verfahren zur Herstellung eines Reinigungs- oder Pflegegeräts (1) nach einem der vorherstehenden Ansprüche, **dadurch gekennzeichnet, dass**
das Reinigungs- oder Pflegegerät (1) mit einer magnetische Kupplungsvorrichtung (9) versehen wird, die zum Bewegen des Rahmens (6) relativ zur zumindest einen Anschlussvorrichtung (4A, 4B, 4C) eine außerhalb des Reinigungs- oder Pflegeraums (3) erzeugte Antriebsbewegung auf den Rahmen (6) überträgt.

15. Verfahren zur Herstellung eines Reinigungs- oder Pflegegeräts (1) nach Anspruch 14, **dadurch gekennzeichnet, dass**
das Reinigungs- oder Pflegegerät (1) mit zumindest zwei Anschlussvorrichtungen (4A, 4B, 4C) für zu reinigende Instrumente (2) versehen wird, die derart relativ zu dem Rahmen (6) bewegbar sind, dass jeweils nur eine der Anschlussvorrichtungen (4A, 4B, 4C) einer Durchbohrung (18) des Rahmens (6) gegenüber steht, an welcher die zumindest eine Düse (7) zur Abgabe eines Reinigungs- oder Pflegemediums vorgesehen ist.

## Claims

1. A cleaning or care device (1) for medical instruments (2), comprising: A cleaning or care space (3), at least one connecting device (4A, 4B, 4C) which is arranged in the cleaning or care space (3) and to which an instrument to be cleaned (2) can be connected, and a delivery device (5) for delivery of at least one cleaning or care medium, wherein the delivery device (5) comprises a frame (6), on which at least one nozzle (7) is provided for dispensing a cleaning or care medium on the outside of the instrument to be cleaned (2), wherein the frame (6) can be moved in relation to the at least one connecting device (4A, 4B, 4C), to substantially follow a longitudinal axis (8) of an instrument (2) connected to the at least one connecting device (4A, 4B, 4C),
**characterized by**
a magnetic coupling device (9), which transmits a driving movement generated outside of the cleaning or care space (3) to the frame (6) for moving the frame (6) in relation to the at least one connecting device (4A, 4B, 4C).

2. The cleaning or care device (1) according to claim 1, **characterized in that**
the magnetic coupling device (9) comprises at least one first magnetic element (11) arranged outside of the cleaning or care space (3) and connected to a drive device (10), which is magnetically coupled to at least one second magnetic element (12) connected to the frame (6).

3. The cleaning or care device (1) according to claim 2, **characterized in that**
the first magnetic element (11) and the second magnetic element (12) are separated from one another by a wall (13) of the cleaning or care space (3).

4. The cleaning or care device (1) according to claim 2 or 3, **characterized in that**
the drive device (10) connected to the first magnetic element (11) comprises a linear drive that moves the first magnetic element (11) along a wall (13) of the cleaning or care space (3).

5. The cleaning or care device (1) according to any one of claims 2 - 4, **characterized in that**
the drive device (10) connected to the first magnetic element (11) comprises a belt drive (14).

6. The cleaning or care device (1) according to any one of the preceding claims,
**characterized in that**
a guide device (15, 15A, 15B) for guiding the frame (6) along the wall (13) of the cleaning or care space (3) is provided on the frame (6) and/or on a wall (13) of the cleaning or care space (3).

7. The cleaning or care device (1) according to any one of the preceding claims,
**characterized in that**
the frame (6) comprises one or more protrusions (17) protruding away from the body (16) of the frame (6), with which the frame (6) slides on a wall (13) of the cleaning or care space (3).

8. The cleaning or care device (1) according to any one of the preceding claims,
**characterized in that**
the body (16) of the frame (6) comprises a through-bore (18), in which an instrument (2) to be cleaned can be accommodated, wherein multiple nozzles (7) are arranged on the self-contained inside wall (19) of the through-bore.

9. The cleaning or care device (1) according to any one of claims 2 - 8, **characterized by** a detection device (20) for detecting a relative movement between the frame (6) and the first magnetic element (11).

10. The cleaning or care device (1) according to claim 9, **characterized in that**
the detection device (20) comprises a magnetic sensor (21), which is movable jointly with the first magnetic element (11) arranged outside of the cleaning or care space (3) and which is operatively connected to a magnetic element (12, 22) provided on the frame (6).

11. The cleaning or care device (1) according to any one of the preceding claims,
**characterized in that**
the at least one connecting device (4A, 4B, 4C) comprises a channel (23) connected to the delivery device (5) for delivery of at least one cleaning or care medium through which a cleaning or care medium can be delivered into the interior of an instrument (2).

12. The cleaning or care device (1) according to any one of the preceding claims,
**characterized in that**
at least two connecting devices (4A, 4B, 4C) are provided for instruments (2) to be cleaned, which are movable relative to the frame (6) such that only one of the connecting devices (4A, 4B, 4C) is opposite a through-bore (18) in the frame (6), on which the at least one nozzle (7) for dispensing a cleaning or care medium is provided.

13. The cleaning or care device (1) according to claim 12, **characterized in that**
the at least two connecting devices (4A, 4B, 4C) are arranged on a shared base element (24), which is arranged rotatably in the cleaning or care device (1).

14. A method for producing a cleaning or care device (1) according to any one of the preceding claims, **characterized in that**
the cleaning or care device (1) is provided with a magnetic coupling device (9), which transfers a driving movement generated outside of the cleaning or care space (3) to the frame (6) for moving the frame (6) relative to the at least one connecting device (4A, 4B, 4C).

15. The method for producing a cleaning or care device (1) according to claim 14,
**characterized in that**
the cleaning or care device (1) is provided with at least two connecting devices (4A, 4B, 4C) for instruments (2) to be cleaned, which are movable relative to the frame (6) such that only one of the connecting devices (4A, 4B, 4C) is opposite a through-bore (18) in the frame (6), on which the at least one nozzle (7) for dispensing a cleaning or care medium is provided.

## Revendications

1. Appareil de nettoyage ou d'entretien (1) pour des instruments médicaux (2), comprenant: un compartiment de nettoyage ou d'entretien (3), au moins un dispositif de raccordement (4A, 4B, 4C), disposé dans le compartiment de nettoyage ou d'entretien (3), auquel il est possible de raccorder un instrument (2) à nettoyer, et un dispositif d'acheminement (5) pour l'acheminement d'au moins un milieu de nettoyage ou d'entretien, le dispositif d'acheminement (5) présentant un cadre (6) sur lequel est prévu au moins une buse (7) pour délivrer un milieu de nettoyage ou d'entretien vers le côté extérieur de l'instrument (2) à nettoyer, le cadre (6) pouvant être déplacé par rapport à cet au moins un dispositif de raccordement (4A, 4B, 4C) afin de suivre sensiblement un axe longitudinal (8) d'un instrument (2) raccordé à cet au moins un dispositif de raccordement (4A, 4B, 4C), **caractérisé par** un dispositif de couplage magnétique (9), lequel transmet au cadre (6) un mouvement d'entraînement généré à l'extérieur du compartiment de nettoyage ou d'entretien (3) pour le déplacement du cadre (6) par rapport à cet au moins un dispositif de raccordement (4A, 4B, 4C).

2. Appareil de nettoyage ou d'entretien (1) selon la revendication 1, **caractérisé en ce que** le dispositif de couplage magnétique (9) présente au moins un premier élément magnétique (11), disposé à l'extérieur du compartiment de nettoyage ou d'entretien (3), en liaison avec un dispositif d'entraînement (10), lequel est couplé magnétiquement avec au moins un deuxième élément magnétique (12) relié au cadre (6).

3. Appareil de nettoyage ou d'entretien (1) selon la revendication 2, **caractérisé en ce que** le premier élément magnétique (11) et le deuxième élément magnétique (12) sont séparés l'un de l'autre par une paroi (13) du compartiment de nettoyage ou d'entretien (3).

4. Appareil de nettoyage ou d'entretien (1) selon la revendication 2 ou 3, **caractérisé en ce que**
le dispositif d'entraînement (10) relié au premier élément magnétique (11) comprend un entraînement linéaire qui déplace le premier élément magnétique (11) le long d'une paroi (13) du compartiment de nettoyage ou d'entretien (3).

5. Appareil de nettoyage ou d'entretien (1) selon l'une des revendications 2 - 4, **caractérisé en ce que**
le dispositif d'entraînement (10) relié au premier élément magnétique (11) présente un entraînement par courroie (14).

6. Appareil de nettoyage ou d'entretien (1) selon l'une des revendications précédentes, **caractérisé en ce que**
l'on prévoit, sur le cadre (6) et/ou sur une paroi (13) du compartiment de nettoyage ou d'entretien (3), un dispositif de guidage (15, 15A, 15B) pour guider le cadre (6) le long de la paroi (13) du compartiment de nettoyage ou d'entretien (3).

7. Appareil de nettoyage ou d'entretien (1) selon l'une des revendications précédentes, **caractérisé en ce que**
le cadre (6) présente un ou plusieurs prolongement(s) (17) dépassant du corps (16) du cadre (6), avec lesquels le cadre (6) coulisse sur une paroi (13) du compartiment de nettoyage ou d'entretien (3).

8. Appareil de nettoyage ou d'entretien (1) selon l'une des revendications précédentes, **caractérisé en ce que**
le corps (16) du cadre (6) présente une perforation (18) dans laquelle un instrument (2) à nettoyer peut être réceptionné, plusieurs buses (7) étant disposées sur la paroi interne fermée (19) de la perforation (18).

9. Appareil de nettoyage ou d'entretien (1) selon l'une des revendications 2 - 8, **caractérisé par**
un dispositif de détection (20) pour la preuve d'un déplacement relatif entre le cadre (6) et le premier élément magnétique (11).

10. Appareil de nettoyage ou d'entretien (1) selon la revendication 9, **caractérisé en ce que**
le dispositif de détection (20) comprend un capteur magnétique (21), lequel peut être déplacé ensemble avec le premier élément magnétique (11) disposé à l'extérieur du compartiment de nettoyage ou d'entretien (3), et lequel est relié de manière opérative à un élément magnétique (12, 22) prévu sur le cadre (6).

11. Appareil de nettoyage ou d'entretien (1) selon l'une des revendications précédentes, **caractérisé en ce que**
cet au moins un dispositif de raccordement (4A, 4B, 4C) présente un canal (23) relié au dispositif d'acheminement (5) pour l'acheminement d'au moins un milieu de nettoyage ou d'entretien, par l'intermédiaire duquel un milieu de nettoyage ou d'entretien peut être acheminé à l'intérieur d'un instrument (2).

12. Appareil de nettoyage ou d'entretien (1) selon l'une des revendications précédentes, **caractérisé en ce que**
l'on prévoit au moins deux dispositifs de raccordement (4A, 4B, 4C) pour des instruments (2) à nettoyer, lesquels peuvent être déplacés par rapport au cadre (6) de manière à ce que respectivement seulement l'un des dispositifs de raccordement (4A, 4B, 4C) se trouve en vis-à-vis d'une perforation (18) du cadre (6), au niveau de laquelle au moins une buse (7) est prévue pour la délivrance d'un milieu de nettoyage ou d'entretien.

13. Appareil de nettoyage ou d'entretien (1) selon la revendication 12, **caractérisé en ce que**
ces au moins deux dispositifs de raccordement (4A, 4B, 4C) sont disposés sur un élément de base (24) commun, lequel est disposé de manière à pouvoir tourner dans l'appareil de nettoyage ou d'entretien (1).

14. Procédé pour la fabrication d'un appareil de nettoyage ou d'entretien (1) selon l'une des revendications précédentes, **caractérisé en ce que**
l'appareil de nettoyage ou d'entretien (1) est muni d'un dispositif de couplage magnétique (9), lequel transmet au cadre (6) un mouvement d'entraînement généré à l'extérieur du compartiment de nettoyage ou d'entretien (3) pour le déplacement du cadre (6) par rapport à cet au moins un dispositif de raccordement (4A, 4B, 4C).

15. Procédé pour la fabrication d'un appareil de nettoyage ou d'entretien (1) selon la revendication 14, **caractérisé en ce que**
l'appareil de nettoyage ou d'entretien (1) est muni d'au moins deux dispositifs de raccordement (4A, 4B, 4C) pour des instruments à nettoyer (2), lesquels peuvent être déplacés par rapport au cadre (6) de manière à ce que respectivement seulement l'un des dispositifs de raccordement (4A, 4B, 4C) se trouve en vis-à-vis d'une perforation (18) du cadre (6) au niveau de laquelle cette au moins une buse (7) pour le déversement d'un milieu de nettoyage ou d'entretien est prévue.
